(19) Europäisches Patentamt
European Patent Office
Office européen des brevets

(11) Numéro de publication : **0 358 571 B1**

# (12) FASCICULE DE BREVET EUROPEEN

(45) Date de publication du fascicule du brevet : **13.01.93 Bulletin 93/02**

(21) Numéro de dépôt : **89402426.4**

(22) Date de dépôt : **06.09.89**

(51) Int. Cl.$^5$ : **C07D 233/42,** C07D 233/84, C07D 239/38, C07D 239/10, C07D 235/28, C07D 249/12

## (54) Thioformamidines, leur préparation et leur application en tant que médicaments.

(30) Priorité : **08.09.88 FR 8811747**

(43) Date de publication de la demande : **14.03.90 Bulletin 90/11**

(45) Mention de la délivrance du brevet : **13.01.93 Bulletin 93/02**

(84) Etats contractants désignés : **AT BE CH DE ES FR GB GR IT LI LU NL SE**

(56) Documents cités :
**FR-A- 1 369 753**
**FR-A- 2 081 483**
**FR-A- 2 344 545**
**US-A- 2 813 102**
**US-A- 3 334 112**
**US-A- 3 715 367**
**US-A- 3 720 686**
**US-A- 3 773 957**
**US-A- 3 920 648**
**PATENT ABSTRACTS OF JAPAN, vol. 9, no. 309 (C-318)[2032], 5 decembre 1985**

(73) Titulaire : **PIERRE FABRE MEDICAMENT**
**45, Place Abel Gance**
**F-92100 Boulogne (FR)**

(72) Inventeur : **Patoiseau, Jean-François**
**7 rue Jules Ferry**
**F--81100 Castres (FR)**
Inventeur : **Autin, Jean-Marie**
**Péri-Albo, Vivier les Montagnes**
**F-81290 Labruguiere (FR)**
Inventeur : **Cousse, Henri**
**La Foun de los Nobios Chemin de Lastinos**
**F-81100 Castres (FR)**
Inventeur : **Sales, Véronique**
**Résidence "La Source" 23 rue Théron Périé**
**F-81100 Castres (FR)**
Inventeur : **Tisne-Versailles, Jacky**
**Chemin de la Barque Quartier de l'Intendant**
**F-81100 Castres (FR)**
Inventeur : **Bali, Jean-Pierre CNRS-LP 84-02**
**Faculté de Pharmacie 15 avenue Charles Flahaut**
**F-34060 Montpellier Cédex (FR)**

(74) Mandataire : **Ahner, Francis et al**
**CABINET REGIMBEAU, 26, avenue Kléber**
**F-75116 Paris (FR)**

EP 0 358 571 B1

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

Jouve, 18, rue Saint-Denis, 75001 PARIS

## Description

La présente invention, réalisée au Centre de Recherches Pierre Fabre, a pour objet de nouveaux composés chimiques, leur procédé de préparation et leur application en tant que médicaments. Les nouveaux composés chimiques, répondent à la formule générale I :

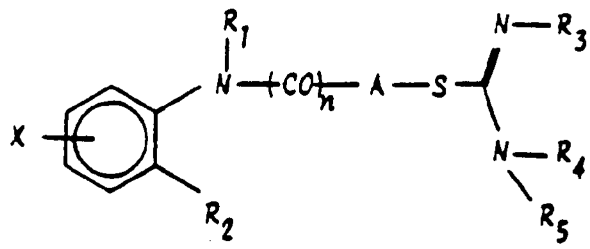

dans laquelle :

- $R_1$ représente l'hydrogène ou un radical alcoyle inférieur contenant de un à quatre atomes de carbone ;
- $R_2$ représente un radical benzoyle, benzyle ou $\alpha$ hydroxy benzyle, le noyau aromatique pouvant être substitué par un atome d'halogène ;
- $R_3$ et $R_4$, identiques ou différents, représentent l'hydrogène, un radical alcoyle ou alcényle inférieur en $C_{1-4}$ ou forment ensemble avec la fonction formamidine ou hétérocycle imidazolé, imidazoline, benzimidazole, triazole, pyrimidine, tétrahydropyrimidine ;
- $R_5$ représente l'hydrogène, un radical alcoyle inférieur contenant de 1 à 4 atomes de carbone ou forme avec $R_4$ une double liaison ($-N=R_4$) dans le cas d'hétérocycles pseudoaromatiques ;
- n = 0 ou 1 ;
- A représente un groupe alcoylène linéaire ou ramifié, comportant de 1 à 4 atomes de carbone ;
- X représente l'hydrogène, un halogène, un alcoyle inférieur en $C_{1-4}$, un alcoxy inférieur en $C_{1-4}$ ou un groupement nitro.

L'invention couvre, en outre, les sels organiques ou minéraux thérapeutiquement acceptables de ces molécules.

La présente invention concerne également un procédé de préparation des dérivés de formule I par réaction de thioformamidines sur un réactif halogéné :

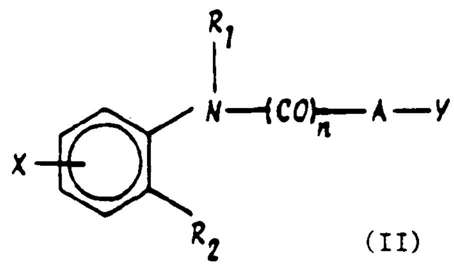

(II)

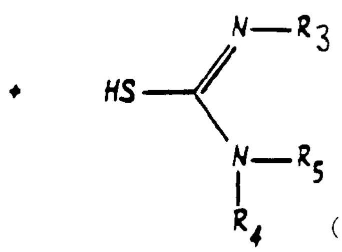

(III)

- Y représente plus particulièrement le chlore ou le brome ;
- $R_1$, $R_2$, $R_3$, $R_4$, $R_5$, A, n et X ayant la même signification que précédemment.

La réaction est conduite de préférence dans un solvant organique tel que méthanol ou éthanol à reflux.

La présente invention concerne de même l'utilisation des composés de formule I à titre de médicaments ainsi que les compositions pharmaceutiques contenant ces médicaments.

Les compositions pharmaceutiques selon la présente invention peuvent comporter un ou plusieurs composés de formule I éventuellement en association avec d'autres principes actifs. Parmi les dérivés de formule I, on peut citer plus particulièrement les composés suivants :

- N-méthyl [($\Delta$2-imidazolinyl)-2 thio]-2 orthochlorobenzoyl-2′ chloro-4′ acétanilide, chlorhydrate, (1)

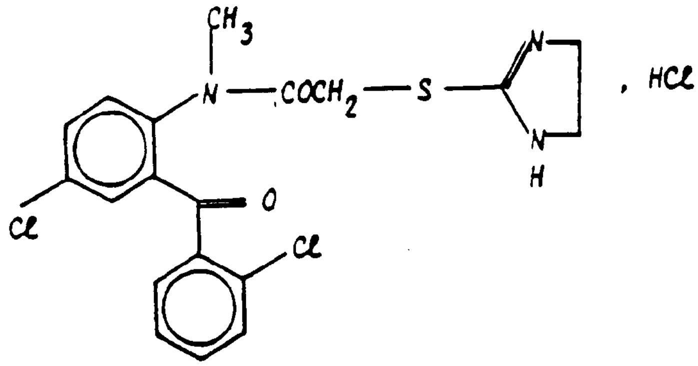

M = 458,79
F° = 202°C

- N-méthyl [(Δ2-imidazolinyl)-2 thio]-2 benzoyl-2′ chloro-4′ acétanilide, chlorhydrate, (2)

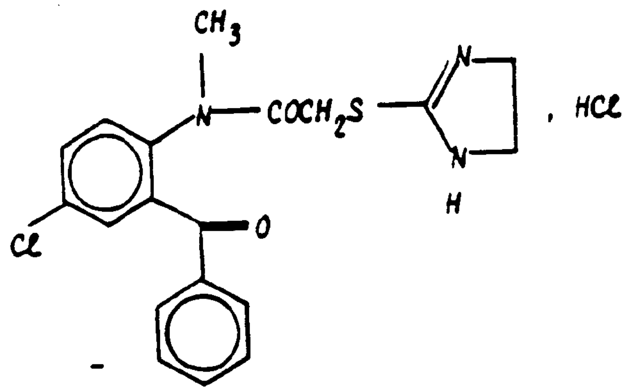

M = 424,35
F° = 202°C

- [(Δ2-imidazolinyl)-2 thio]-2 orthochlorobenzoyl-2′ chloro-4′ acétanilide, chlorhydrate, (3)

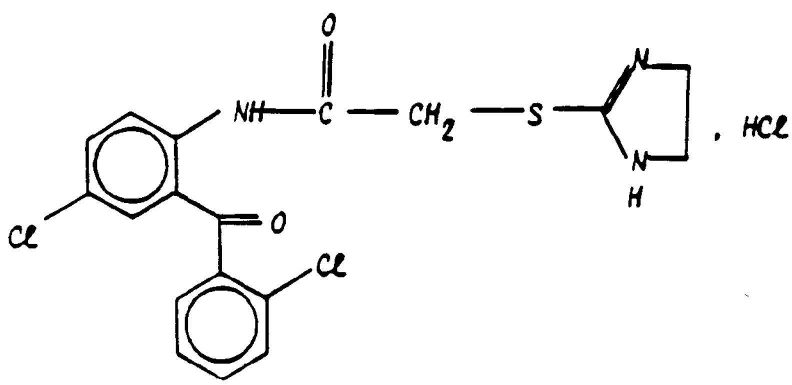

M = 444,76
F° = 184°C

- N-méthyl [(méthyl-1 imidazolyl)-2 thio]-2 orthochlorobenzoyl-2′ chloro-4′ acétanilide, chlorhydrate, (4)

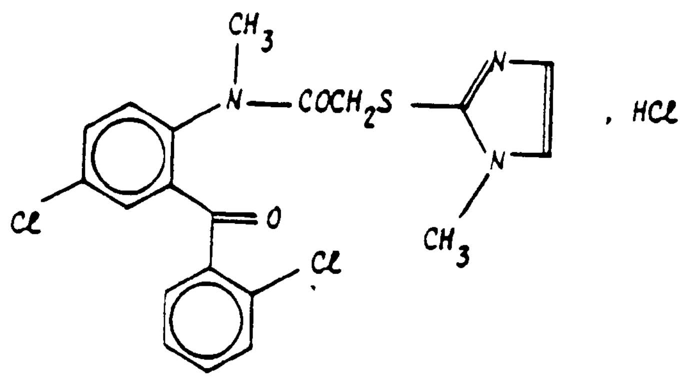

M = 470,8
F° = 186°C

- (imidazolyl-2 thio)-2 benzoyl-2′ chloro-4′ acétanilide, chlorhydrate, (5)

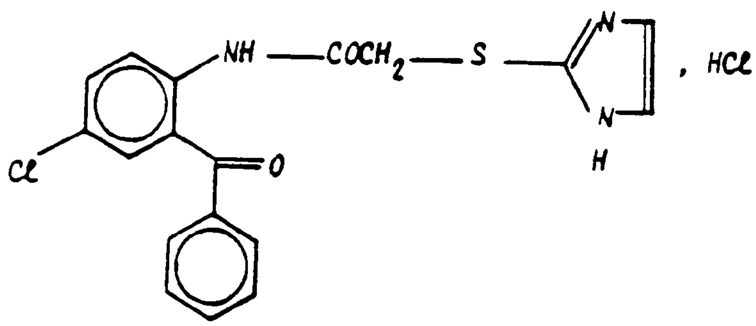

M = 408,31
F° = 194°C

- (imidazolyl-2 thio)-2 orthochlorobenzoyl-2′ chloro-4′ acétanilide, chlorhydrate, (6)

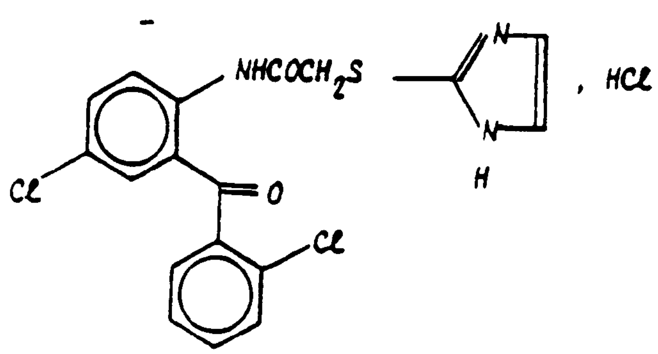

M = 442,75
F° = 178°C

- N-méthyl o.chlorobenzoyl-2′ chloro-4′ [(imidazolyl-2) thio]-2 acétanilide, chlorhydrate, (7)

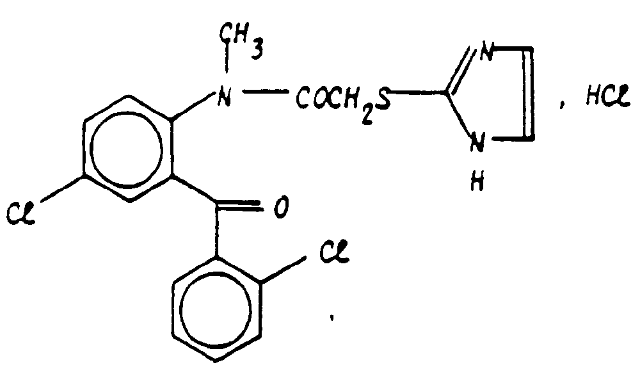

M = 456,78
F° = 165°C

- (imidazolyl-2 thio)-1 [(benzoyl-2 chloro-4) anilino]-2 éthane, (8)

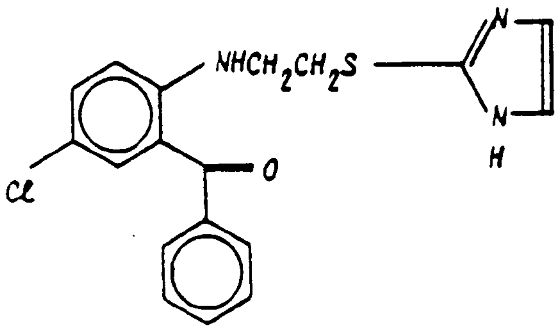

M = 357,86
F° = 203°C

- [(Δ2-imidazolinyl)-2 thio]-1 (benzoyl-2 chloro-4) anilino-2 éthane, (9)

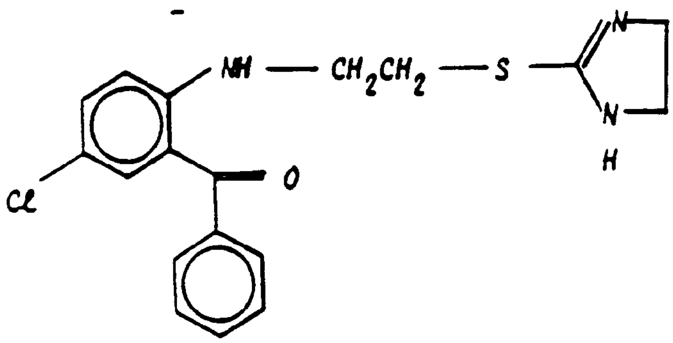

M = 359,87
F° = 146°C

- N-méthyl o.chlorobenzoyl-2′ chloro-4′ [(méthyl-1 Δ2-imidazolinyl)-2 thio]-2 acétanilide, chlorhydrate, (10)

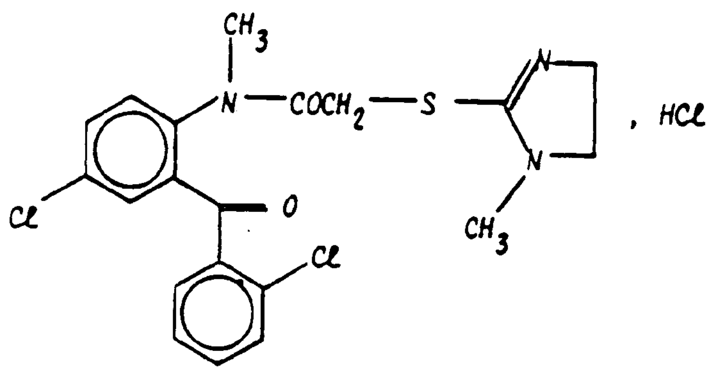

M = 472,83
F° = 185°C

- N-méthyl (orthochloro α-hydroxybenzyl)-2′ chloro-4′ [(Δ2-imidazolinyl)-2 thio]-2 acétanilide, (11)

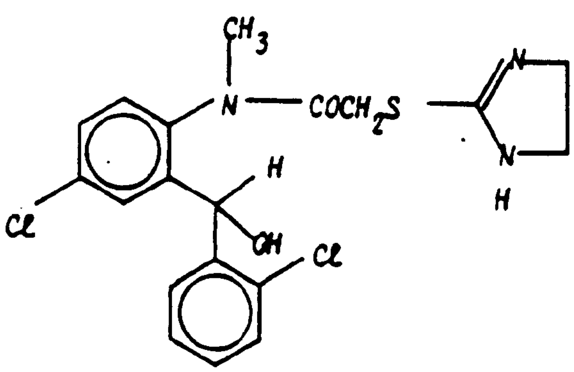

M = 424,35
F° = 170°C

- N-méthyl benzyl-2′ chloro-4′ [(Δ2-imidazolinyl)-2 acétanilide, chlorhydrate, (12)

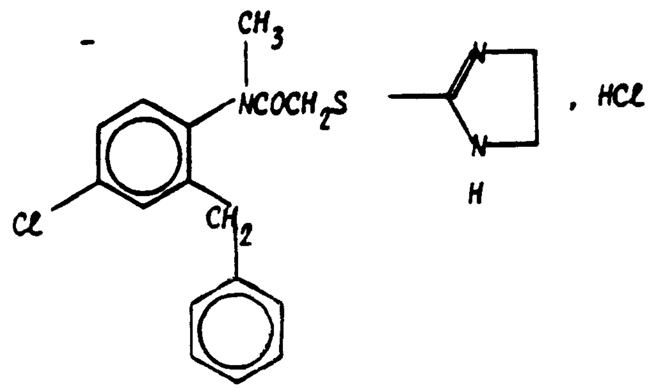

M = 410,35
F° = 217°C

- N-méthyl orthochlorobenzoyl-2′ chloro-4′ [(Δ2-imidazolinyl)-2 thio]-2 propionanilide, citrate, (13)

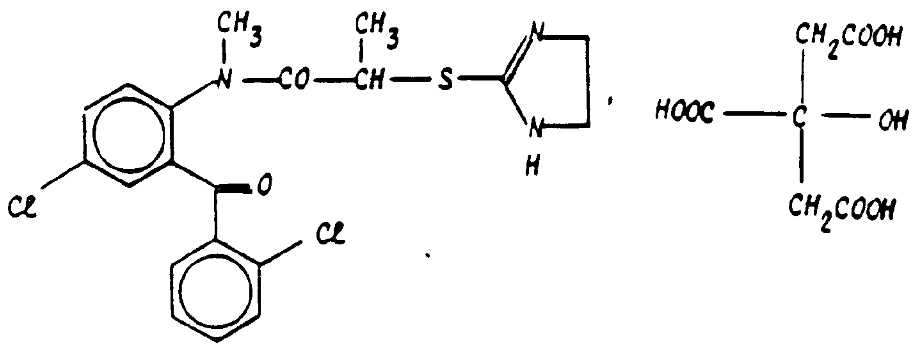

M = 628,49
F° = 92°C

- N-méthyl benzoyl-2′ [imidazolyl-2 thio]-2 acétanilide, (14)

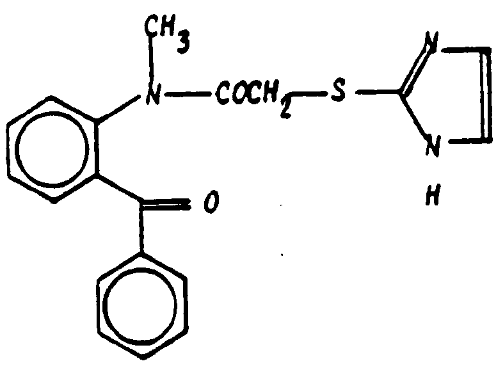

M = 351,43
F° = 107°C

- N-méthyl benzoyl-2′ chloro-4′ (imidazolyl-2 thio)-2 acétanilide, chlorhydrate, (15)

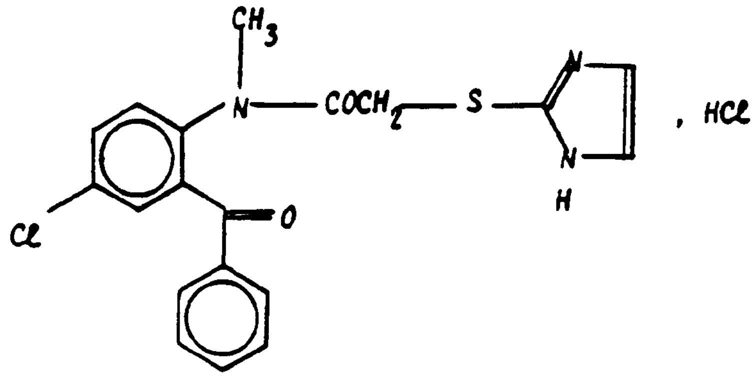

M = 422,33
F° = 180°C

- N-méthyl orthochlorobenzoyl-2′ chloro-4′ isothiouréido-2 acétanilide, chlorhydrate, (16)

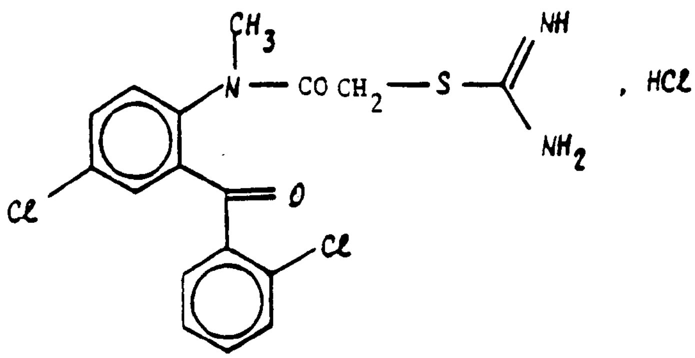

M = 432,76
F° = 195°C

- N-méthyl orthochlorobenzoyl-2′ chloro-4′ (pyrimidinyl-2) thio-2 acétanilide, (17)

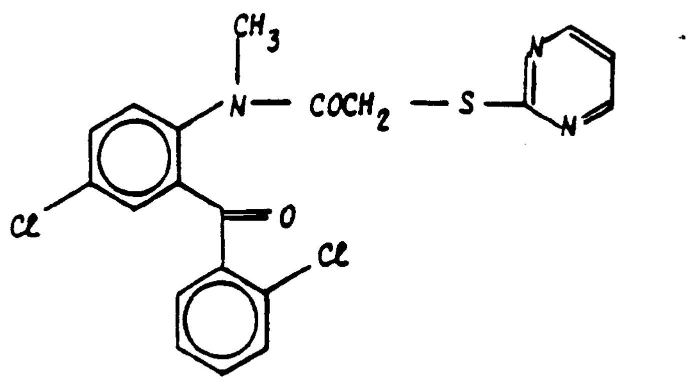

M = 432,33
F° = 138°C

- N-méthyl orthochlorobenzoyl-2′ chloro-4′ (éthyl-1 imidazolyl-2) thio-2 acétanilide, chlorhydrate, (18)

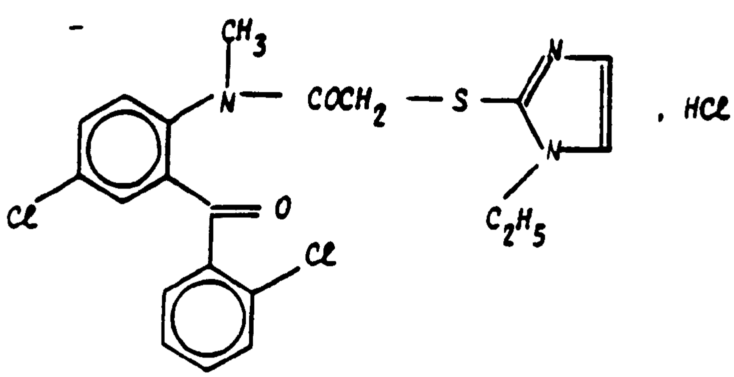

M = 484,83
F° = 172°C

- N-méthyl orthochlorobenzoyl-2′ chloro-4′ (imidazolyl-2) thio-3 propionanilide, (19)

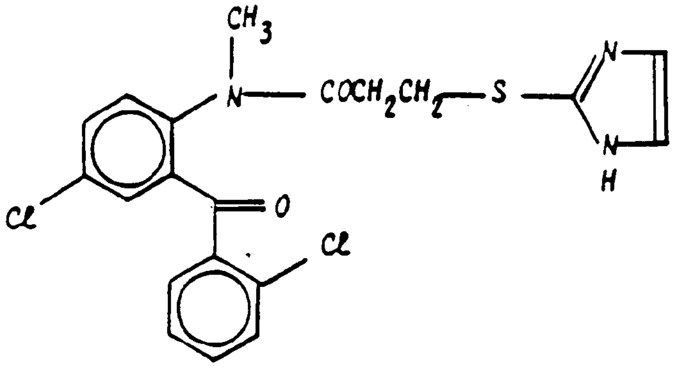

M = 434.34
F° = 95°C

L'invention sera décrite ci-après plus en détail à travers les quelques exemples suivants :
- N-méthyl benzoyl-2′ nitro-4′ [(imidazolyl-2) thio]-2 acétanilide, chlorhydrate (20)

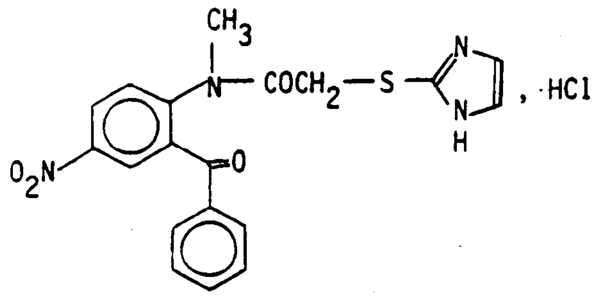

M = 432.88
F = 205°C

- N-méthyl o.fluorobenzoyl-2′ chloro-4′ [(imidazolyl-2) thio]-2 acétanilide, chlorhydrate (21)

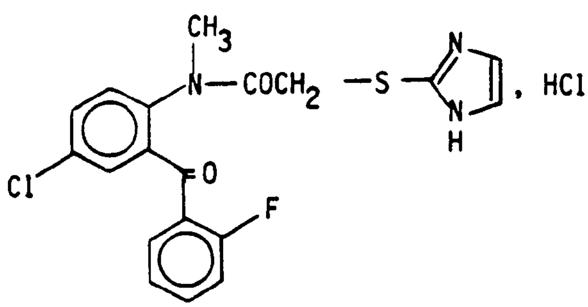

M = 440,12
F = 166°C

- N-méthyl benzoyl-2′ méthoxy-4′ [(imidazolyl-2) thio]-2 acétanilide, bromhydrate (22)

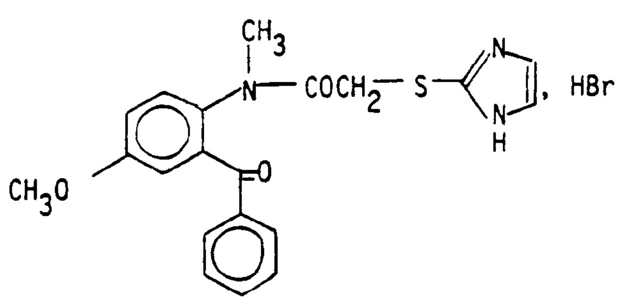

M = 462,37
F = 128°C

Exemple 1 :

Préparation de la N-méthyl [(Δ2-imidazolinyl)-2 thio]-2 benzoyl-2′ chloro-4′ acétanilide, chlorhydrate, (2)

22,55 g de N-méthylbenzoyl-2′dichloro-2,4′acétanilide et 6,13 g d'éthylène thiourée sont portés 6 heures à reflux dans 150 ml d'éthanol absolu. La solution est évaporée sous vide et reprise dans un minimum de chloroforme et diluée à l'éther éthylique. On obtient par cristallisation 23,6 g de N-méthyl [(Δ2-imidazolinyl)-2 thio]-2 benzoyl-2′ chloro-4′ acétanilide, chlorhydrate (Rdt = 92 %).
F° = 202°C
C.C.M. = gel de silice 60 F 254 Merck - Solvant : $CHCl_3$-MeOH : 85-15 - Rf = 0,27.

Exemple 2 :

N-méthyl o.chlorobenzoyl-2′ chloro-4′ [(imidazolyl-2) thio]-2 acétanilide, chlorhydrate, (7)

Une suspension de 10,68 g de N-méthyl orthochlorobenzoyl-2′-dichloro-2,4′acétanilide et de 2,4 g de mercapto-2 imidazole sont traités pendant 8 heures au reflux du méthanol. Après refroidissement, évaporation sous vide et reprise par un mélange acétone-éther, on obtient par cristallisation le chlorhydrate de N-méthylorthochlorobenzoyl-2′chloro-4′ (imidazolyl-2) thio-2 acétanilide (9,12 g).
F° = 165°C
Rf = 0,22 (acétate d'éthyle)

Exemple 3

[(Δ2-imidazolinyl)-2 thio]-1 (benzoyl-2 chloro-4) anilino-2 éthane, (9)

Une suspension de 16,9 g de β bromo éthylamino-2 chloro-5 benzophénone et de 5 g d'éthylène thiourée est portée 3 heures au reflux d'une solution contenant 100 ml d'éthanol 95°, 10 ml de soude 10N et 15 ml d'eau. Aprés évaporation sous vide, reprise à l'acide chlorhydrique 6N et lavage à l'acétate d'éthyle, la phase aqueuse est neutralisée au bicarbonate de sodium et extraite à l'acétate d'éthyle. La phase organique, concentrée, est purifiée sur colonne de silice par élution MeOH-$CHCl_3$-$NH_4OH$ : 9-90-1 et l'on isole par cristallisation dans l'acétate d'éthyle le [(Δ2-imidazolin-yl)-2 thio]-1 (benzoyl-2 chloro-4) anilino-2 éthane ;
F° = 146°C
Rf = 0,13 ($CHCl_3$-MeOH : 85-15)

Exemple 4

N-méthyl (orthochloro α-hydroxybenzyl)-2′ chloro-4′ [(Δ2-imidazolinyl)-2 thio]-2 acétanilide, (11)

A une suspension de 2,8 g de méthylamino-2 dichloro-2′,5 benzophénone dans 20 ml de méthanol, on ajoute par portions 750 mg de borohydrure de sodium. Après une dizaine de minutes de réaction, le solvant est évaporé sous vide. Après reprise à l'éther, lavage à l'eau puis séchage, on isole par cristallisation le méthylamino-2 dichloro-2′,5 benzhydrol (2,52 g).
F° = 110°C.
Le composé obtenu (2,26 g) en solution dans l'acétate d'éthyle est traité à une température de 0°C par 0,8 ml de chlorure de bromoacétyle en solution dans l'acétate d'éthyle (10ml). Après lavage à l'eau et séchage, on obtient le (N-méthyl bromoacétamido)-2 dichloro-2′,5 benzhydrol (2,83 g). F° = 169°C.
Ce produit (6,5 g) en suspension dans l'éthanol absolu (30 ml) est traité par l'éthylène thiourée (1,63 g) au reflux pendant 3 heures. Après refroidissement, on obtient des cristaux qui sont filtrés et lavés à l'éther. Par reprise à la soude N, extraction par THF-acétate d'éthyle puis séchage et recristallisation dans l'acétate d'éthyle, on obtient le N-méthyl (orthochloro α-hydroxy benzyl)-2′ chloro-4′ [(Δ2-imidazolinyl)-2 thio]-2 acétanilide.
F° = 170°C
Rf = 0,2 ($CHCl_3$-MeOH : 85-15)

Exemple 5 :

N-méthyl benzyl 2′ chloro-4′ [(Δ2-imidazolinyl)-2 thio]-2 acétanilide, chlorhydrate, (12)

a) Benzyl-2′ chloro-4′ tosanilide

A une solution de benzyl-2 chloro-4 aniline (7.9 g) dans la pyridine (35 ml), on ajoute 8.31 g de chlorure de tosyle. Après 1 heure à 100°C puis refroidissement, on jette sur 100 ml d'acide chlorhydrique 6N et 200 g de glace. Il se forme une gomme qui est extraite à l'acétate d'éthyle. Après lavage à l'eau, séchage et concentration, on obtient par précipitation à l'éther de pétrole, le benzyl-2′ chloro-4′ tosanilide (10,85 g). F° = 115°C

b) N-méthyl benzyl-2′ chloro-4′ tosanilide

Le composé ci-dessus (8,82 g) en solution dans le toluène est additionné de chlorure de benzyltriéthylammonium (522 mg) soude 10N (23,6 ml) et de sulfate de méthyle (5 ml). Après 30 mn sous agitation et abandon une nuit, le mélange est décanté, lavé à l'eau puis séché sur $Na_2SO_4$, on obtient par cristallisation le N-méthyl benzyl-2′ chloro-4′ tosanilide (8,37 g).
F° = 114°C

c) N-méthylbenzyl-2chloro-4aniline

Le produit précédent (24,49) en suspension dans l'acide acétique (50 ml) et l'acide sulfurique 70 % (50 ml) est porté 11 heures à 100°C puis jeté sur 100 cc d'eau glacée. Après neutralisation à la soude 10N, extraction à l'éther, lavage à l'eau et séchage, on obtient par évaporation une huile orangée (13,16 g), chlorhydrate.
F° = 125°C

d) N-méthyl benzyl-2′ chloro-4′ bromacétanilide

La N-méthylbenzyl-2chloro-4aniline (10,8 g) en solution dans l'acétate d'éthyle est traitée à 5°C par 4,9 ml de bromure de bromoacétyle. Après 1 h à température ambiante, décantation et lavage à l'eau, la solution est concentrée. On obtient après cristallisation la N-méthyl benzyl-2′chloro-4′bromacétanilide (12.21 g).
F° = 78°C

e) Chlorhydrate de N-méthyl benzyl-2′ chloro-4′ [(Δ2-imidazolin yl)-2 thio]-2 acétanilide

Le produit obtenu ci-dessus (1.76 g) est traité au reflux de l'éthanol (20 ml) pendant 2 heures en présence d'éthylène thiourée. Après refroidissement et filtration, on obtient 2,19 g de produit brut. On effectue une reprise à la soude N, extraction à l'acétate d'éthyle, lavage à l'eau, puis évaporation sous vide. Le résidu, traité par une solution éthanolique d'acide chlorhydrique, donne par cristallisation dans l'éther éthylique le chlorhydrate de N-méthyl benzyl-2′ chloro-4′ [(Δ2-imidazolinyl)-2 thio]-2 acétanilide (1,7 g).
F° = 217°C.
Rf = 0,7 ($CHCl_3$-MeOH : 50-50).

EXPERIMENTATIONS

Divers essais toxicologiques et pharmacologiques ont été effectués.

A - TOXICOLOGIE

Les composés de l'invention ont été soumis à des contrôles de toxicité. Celle-ci a été déterminée par la dose létale 50 % ($DL_{50}$). Elle a été recherchée sur des lots de 10 souris par voie orale et calculée selon la méthode de Thomson et Weil (Biometrics, 1952, 8, 249). Les $DL_{50}$ des composés testés sont supérieures à 500 mg/kg par voie orale.

B - PROPRIETES PHARMACOLOGIQUES

Les expérimentations pharmacologiques ont permis de mettre en évidence de remarquables propriétés

antiulcéreuses et antisécrétoires gastriques. Ci-après sont reportés, à titre d'exemple, les résultats obtenus avec certains produits de la présente invention comparativement à la cimétidine.

1. Test d'étude de la secrétion gastrite par ligature du pylore selon la technique de Shay (Gastroenterology 5, 1945 et 26, 906, 1954) sur rats Sprague Dawley mâles. Traitements aigu par voie intraduodénale, à la dose de 50 mg/kg.

| Produit | % variation | |
|---|---|---|
| | diminution du volume sécrété | élévation du pH intragastrique |
| Cimétidine | 47 | 90 |
| 1 | 76 | 158 |
| 7 | 70 | 118 |
| 4 | 76 | 267 |
| 16 | 79 | 153 |
| 15 | 50 | 181 |

2. Activité vis-à-vis d'ulcères à l'acide salicylique quantifiée selon la cotation de Pfeiffer (Arch. Int. Pharmacodyn. 190, 5, 13, 1971) sur rats mâles. Traitement par voie orale, en aigu.

| Produit | Dose (mg/kg) | % inhibition des lésions ulcéreuses |
|---|---|---|
| Cimétidine | 40 | 46 |
| 1 | 40 | 34 |
| 7 | 40 | 46 |
| 12 | 40 | 40 |
| 15 | 50 | 35 |
| 16 | 50 | 32 |

C - APPLICATIONS THERAPEUTIQUES

Compte tenu de leurs propriétés pharmacologiques, les composés de l'invention peuvent être utilisés dans le traitement de la pathologie digestive et plus particulièrement de la pathologie ulcéreuse. Les préparations pharmaceutiques contenant ces principes actifs peuvent être administrées par voie orale. Il est également possible d'y associer d'autres principes actifs pharmaceutiquement et thérapeutiquement acceptables.

## Revendications

### Revendications pour les Etats contractants suivants : AT, BE, CH, DE, FR, GB, IT, LI, LU, NL, SE

**1.** Les thioformamidines répondant à la formule générale I :

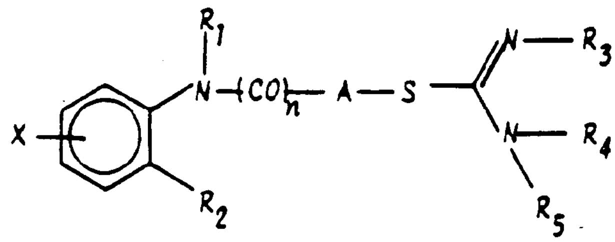

dans laquelle :
- $R_1$ représente l'hydrogène ou un radical alcoyle inférieur contenant de un à quatre atomes de carbone ;
- $R_2$ représente un radical benzoyle, benzyle ou α hydroxy benzyle, le noyau aromatique pouvant être substitué par un atome d'halogène ;
- $R_3$ et $R_4$, identiques ou différents, représentent l'hydrogène, un radical alcoyle ou alcenyle inférieur en $C_{1-4}$, ou forment entre eux avec la fonction formamidine un hétérocycle imidazolé, imidazoline, benzimidazole, triazole, pyrimidine, tétrahydropyrimidine ;
- $R_5$ représente l'hydrogène, un radical alcoyle inférieur contenant de 1 à 4 atomes de carbone ou forme avec $R_4$ une double liaison ($-N=R_4$) dans le cas d'hétérocycles pseudoaromatiques ;
- n = 0 ou 1 ;
- A représente un group alcoylène linéaire ou ramifié, comportant de 1 à 4 atomes de carbone ;
- X représente l'hydrogène, un halogène, un alcoyle inférieur en $C_{1-4}$, un alcoxy inférieur en $C_{1-4}$ ou un groupement nitro,

et leurs sels organiques ou minéraux thérapeutiquement acceptables.

**2.** Un composé selon la revendication 1 caractérisé en ce qu'il est choisi parmi :
- N-méthyl [(Δ2-imidazolinyl)-2 thio]-2 orthochlorobenzoyl-2′ chloro-4′ acétanilide, chlorhydrate ;
- N-méthyl [(Δ2-imidazolinyl)-2 thio]-2 benzoyl-2′ chloro-4′ acétanilide, chlorhydrate ;
- [(Δ2-imidazolinyl)-2 thio]-2 orthochlorobenzoyl-2′ chloro-4′ acétanilide, chlorhydrate ;
- N-méthyl [(méthyl-1 imidazolyl)-2 thio]-2 orthochlorobenzoyl-2′ chloro-4′ acétanilide, chlorhydrate ;
- (imidazolyl-2 thio)-2 benzoyl-2′ chloro-4′ acétanilide, chlorhydrate ;
- (imidazolyl-2 thio)-2 orthochlorobenzoyl-2′ chloro-4′ acétanilide, chlorhydrate ;
- N-méthyl o.chlorobenzoyl-2′ chloro-4′ [(imidazolyl-2) thio]-2 acétanilide, chlorhydrate ;
- (imidazolyl-2 thio)-1 [(benzoyl-2 chloro-4) anilino]-2 éthane ;
- [(Δ2-imidazolinyl)-2 thio]-1 (benzoyl-2 chloro-4) anilino-2 éthane ;
- N-méthyl o.chlorobenzoyl-2′ chloro-4′ [(méthyl-1 Δ2-imidazolinyl)-2 thio]-2 acétanilide, chlorhydrate ;
- N-méthyl (orthochloro α-hydroxybenzyl)-2′ chloro-4′ [Δ2-imidazolinyl)-2 thio]-2 acétanilide ;
- N-méthyl benzyl-2′ chloro-4′ [(Δ2-imidazolinyl)-2 thio]-2 acétanilide, chlorhydrate ;
- N-méthyl orthochlorobenzoyl-2′ chloro-4′ [(Δ2-imidazolinyl)-2 thio]-2 propionanilide, citrate ;
- N-méthyl benzoyl-2′ [imidazolyl-2 thio]-2 acétanilide ;
- N-méthyl benzoyl-2′ chloro-4′ (imidazolyl-2 thio)-2 acétanilide, chlorhydrate ;
- N-méthyl orthochlorobenzoyl-2′ chloro-4′ isothioureido-2 acétanilide, chlorhydrate ;
- N-méthyl orthochlorobenzoyl-2′ chloro-4′ (pyrimidinyl-2) thio-2 acétanilide ;
- N-méthyl orthochlorobenzoyl-2′ chloro-4′ (éthyl-1 imidazolyl-2) thio-2 acétanilide, chlorhydrate ;
- N-méthyl orthochlorobenzoyl-2′ chloro-4′ (imidazolyl-2) thio-3 propionanilide.
- N-méthyl benzoyl-2′ nitro-4′ [(imidazolyl-2) thio]-2 acétanilide, chlorhydrate ;
- N-méthyl o.fluorobenzoyl-2′ chloro-4′ [(imidazolyl-2) thio]-2 acétanilide, chlorhydrate ;
- N-méthyl benzoyl-2′ méthoxy-4′ [(imidazolyl-2) thio]-2 acétanilide, bromhydrate.

**3.** Un procédé de préparation des composés chimiques selon l'une des revendications 1 et 2 caractérisé en ce que l'on traite un dérivé halogéné II par une thioformamidine III au reflux d'un solvant organique tel

que méthanol ou éthanol.

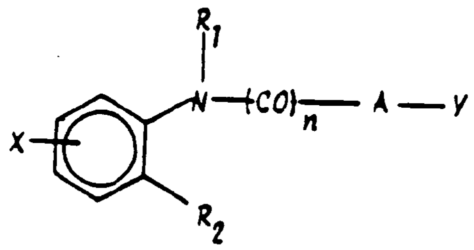

II

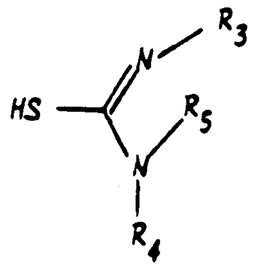

III

$R_1$, $R_2$, $R_3$, $R_4$, $R_5$, A, n et X ont la même signification que précédemment en Y représente un halogène tel que chlore ou brome.

**4.** A titre de médicaments utiles en thérapeutique humaine ou vétérinaire, les composés selon l'une des revendications 1 et 2.

**5.** A titre de médicaments utiles dans le traitement de l'ulcère et de la pathologie gastro-intestinale, les composés selon l'une des revendications 1 et 2.

**6.** Compositions pharmaceutiques caractérisées en ce qu'elles contiennent comme principe actif au moins un dérivé selon l'une des revendications 1 et 2, associé à un support pharmaceutiquement acceptable.

**Revendications pour l'Etat contractant suivant : ES**

**1.** Procédé de préparation de thioformamidines répondant à la formule générale I :

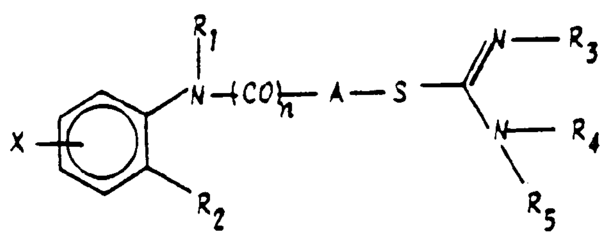

dans laquelle
- $R_1$ représente l'hydrogène ou un radical alcoyle inférieur contenant de un à quatre atomes de carbone;
- $R_2$ représente un radical benzoyle, benzyle ou $\alpha$ hydroxy benzyle, le noyau aromatique pouvant être substitué par un atome d'halogène;
- $R_3$ et $R_4$, identiques ou différents, représentent l'hydrogène, un radical alcoyle ou alcenyle inférieur en $C_{1-4}$, ou forment entre eux avec la fonction formamidine un hétérocycle imidazolé, imidazoline, benzimidazole, triazole, pyrimidine, tétrahydropyrimidine;
- $R_5$ représente l'hydrogène, un radical alcoyle inférieur contenant de 1 à 4 atomes de carbone ou forme avec $R_4$ une double liaison ($-N=R_4$) dans le cas d'hétérocycles pseudoaromatiques;
- n= 0 ou 1;
- A représente un groupe alcoylène linéaire ou ramifié, comportant de 1 à 4 atomes de carbone;
- X représente l'hydrogène, un halogène, un alcoyle inférieur en $C_{1-4}$, un alcoxy inférieur en $C_{1-4}$ ou un groupement nitro, et de leurs sels organiques ou minéraux théapeutiquement acceptables, caractérisé

en ce que l'on traite un dérivé halogéné II par une thioformamidine III au reflux d'un solvant organique tel que méthanol ou éthanol.

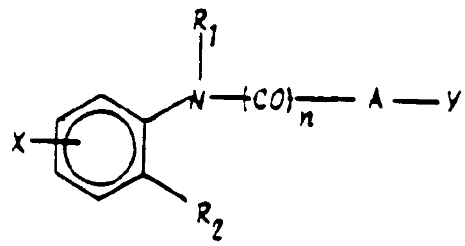

II

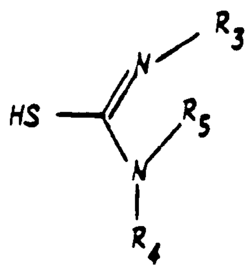

III

$R_1$, $R_2$, $R_3$, $R_4$, $R_5$, A, n et X ont la même signification que précédemment et Y représente un halogène tel que chlore ou brome.

**2.** Procédé selon la revendication 1, caractérisé en ce que l'on prépare un composé choisi parmi :
- N-méthyl [(Δ2-imidazolinyl)-2 thio]-2- orthochlorobenzoyl-2′ chloro-4′ acétanilide, chlorhydrate ;
- N-méthyl [(Δ2-imidazolinyl)-2- thio]-2- benzoyl-2′ chloro-4′ acétanilide, chlorhydrate ;
- [(Δ2-imidazolinyl)-2 thio]-2 orthochlorobenzoyl-2′ chloro-4′ acétanilide, chlorhydrate ;
- N-méthyl [(méthyl-1 imidazolyl)-2 thio]-2 orthochlorobenzoyl-2′ choro-4′ acétanilide, chlorhydrate ;
- (imidazolyl-2 thio)-2 benzoyl-2′ chloro-4′ acétanilide, chlorhydrate ;
- (imidazolyl-2 thio)-2 orthochlorobenzoyl-2′ chloro-4′ acétanilide, chlorhydrate ;
- N-méthyl o.chlorobenzoyl-2′ chloro-4′ [(imidazolyl-2) thio]-2 acétanilide, chlorhydrate ;
- (imidazolyl-2 thio)-1 [(benzoyl-2 chloro-4) anilino]-2 éthane ;
- [(Δ2-imidazolyl)-2 thio]-1 (benzoyl-2 chloro-4) anilino-2 éthane ;
- N-méthyl o.chlorobenzoyl-2′ chloro-4′ [méthyl-1 Δ2-imidazolinyl)-2 thio]-2 acétanilide, chlorohydrate ;
- N-méthyl (orthochloro α-hydroxybenzyl)-2′ chloro-4′ [(Δ2-imidazolinyl) 2 thio]-2 acétanilide ;
- N-méthyl benzyl-2′ chloro-4′ [(Δ2-imidazolinyl)-2 thio]-2 acétanilide, chlorhydrate ;
- N-méthyl orthochlorobenzoyl-2′ chloro-4′ [(Δ2-imidazolinyl)-2 thio]-2 propionanilide, citrate ;
- N-méthyl benzoyl-2′ [imidazolyl-2 thio]-2 acétanilide ;
- N-méthyl benzoyl-2′ chloro-4′ (imidazolyl-2 thio)-2 acétanilide, chlorhydrate ;
- N-méthyl orthochlorobenzoyl-2′ chloro-4′ isothioureido-2 acétanilide, chlorhydrate ;
- N-méthyl orthochlorobenzoyl-2′ chloro-4′ (pyrimidinyl-2) thio-2 acétanilide ;
- N-méthyl orthochlorobenzoyl-2′ chloro-4′ (éthyl-1 imidazolyl-2) thio-2 acétanilide, chlorohydrate ;
- N-méthyl orthochlorobenzoyl-2′ chloro-4′ (imidazolyl-2) thio-3 propionanilide.
- N-méthyl benzoyl-2′ nitro-4′ [(imidazolyl-2) thio]-2 acétanilide, chlorohydrate ;
- N-méthyl o.fluorobenzoyl-2′ chloro-4′ [(imidazolyl-2) thio]-2 acétanilide, chlorohydrate ;
- N-méthyl benzoyl-2′ méthoxy-4′ [imidazolyl-2) thio]-2 acétanilide, bromhydrate.

**Revendications pour l'Etat contractant suivant : GR**

**1.** Les thioformamidines répondant à la formule générale I :

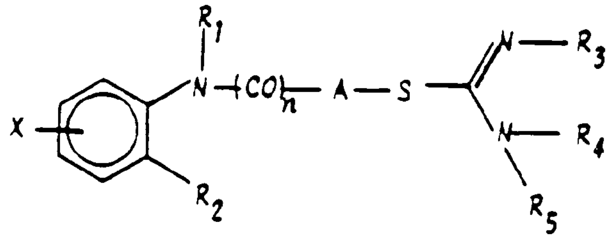

dans laquelle :
- $R_1$ représente l'hydrogène ou un radical alcoyle inférieur contenant de un à quatre atomes de carbone ;
- $R_2$ représente un radical benzoyle, benzyle ou α hydroxy benzyle, le noyau aromatique pouvant être substitué par un atome d'halogène ;
- $R_3$ et $R_4$, identiques ou différents, représentent l'hydrogène, un radical alcoyle ou alcenyle inférieur en $C_{1-4}$, ou forment entre eux avec la fonction formamidine un hétérocycle imidazole, imidazoline, benzimidazole, triazole, pyrimidine, tétrahydropyrimidine ;
- $R_5$ représente l'hydrogène, un radical alcoyle inférieur contenant de 1 à 4 atomes de carbone ou forme avec $R_4$ une double liaison ($-N=R_4$) dans le cas d'hétérocycles pseudoaromatiques ;
- n = 0 ou 1 ;
- A représente un group alcoylène linéaire ou ramifié, comportant de 1 à 4 atomes de carbone ;
- X représente l'hydrogène, un halogène, un alcoyle inférieur en $C_{1-4}$, un alcoxy inférieur en $C_{1-4}$ ou un groupement nitro,

et leurs sels organiques ou minéraux thérapeutiquement acceptables.

2. Un composé selon la revendication 1 caractérisé en ce qu'il est choisi parmi :
- N-méthyl [(Δ2-imidazolinyl)-2 thio]-2 orthochlorobenzoyl-2′ chloro-4′ acétanilide, chlorhydrate ;
- N-méthyl [(Δ2-imidazolinyl)-2 thio]-2 benzoyl-2′ chloro-4′ acétanilide, chlorhydrate ;
- [(Δ2-imidazolinyl)-2 thio]-2 orthochlorobenzoyl-2′ chloro-4′ acétanilide, chlorhydrate ;
- N-méthyl [(méthyl-1 imidazolyl)-2 thio]-2 orthochlorobenzoyl-2′ chloro-4′ acétanilide, chlorhydrate ;
- (imidazolyl-2 thio)-2 benzoyl-2′ chloro-4′ acétanilide, chlorhydrate ;
- (imidazolyl-2 thio)-2 orthochlorobenzoyl-2′ chloro-4′ acétanilide, chlorhydrate ;
- N-méthyl o.chlorobenzoyl-2′ chloro-4′ [(imidazolyl-2) thio]-2 acétanilide, chlorhydrate ;
- (imidazolyl-2 thio)-1 [(benzoyl-2 chloro-4) anilino]-2 éthane ;
- [(Δ2-imidazolinyl)-2 thio]-1 (benzoyl-2 chloro-4) anilino-2 éthane ;
- N-méthyl o.chlorobenzoyl-2′ chloro-4′ [méthyl-1 Δ2-imidazolinyl)-2 thio]-2 acétanilide, chlorhydrate ;
- N-méthyl (orthochloro α-hydroxybenzyl)-2′ chloro-4′ [(Δ2-imidazolinyl)-2 thio]-2 acétanilide ;
- N-méthyl benzyl-2′ chloro-4′ [(Δ2-imidazolinyl)-2 thio]-2 acétanilide, chlorhydrate ;
- N-méthyl orthochlorobenzoyl-2′ chloro-4′ [Δ2-imidazolinyl)-2 thio]-2 propionanilide, citrate ;
- N-méthyl benzoyl-2′ [imidazolyl-2 thio]-2 acétanilide ;
- N-méthyl benzoyl-2′ chloro-4′ (imidazolyl-2 thio)-2 acétanilide, chlorhydrate ;
- N-méthyl orthochlorobenzoyl-2′ chloro-4′ isothioureido-2 acétanilide, chlorhydrate ;
- N-méthyl orthochlorobenzoyl-2′ choro-4′ (pyrimidinyl-2) thio-2 acétanilide ;
- N-méthyl orthochlorobenzoyl-2′ choro-4′ (éthyl-1 imidazolyl-2) thio-2 acétanilide, chlorhydrate ;
- N-méthyl orthochlorobenzoyl-2′ choro-4′ (imidazolyl-2) thio-3 propionanilide.
- N-méthyl benzoyl-2′ nitro-4′ [(imidazolyl-2) thio]-2 acétanilide, chlorhydrate ;
- N-méthyl o.fluorobenzoyl-2′ chloro-4′ [(imidazolyl-2) thio]-2 acétanilide, chlorhydrate ;
- N-méthyl benzoyl-2′ méthoxy-4′ [(imidazolyl-2) thio]-2 acétanilide, bromhydrate.

3. Un procédé de préparation des composés chimiques selon l'une des revendications 1 et 2 caractérisé en ce que l'on traite un dérivé halogéné II par une thioformamide III au reflux d'un solvant organique tel que méthanol ou éthanol.

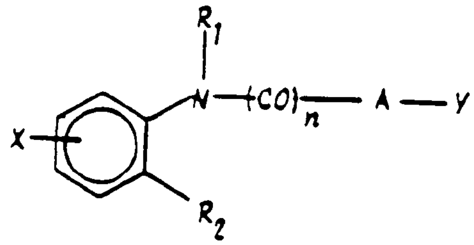

II

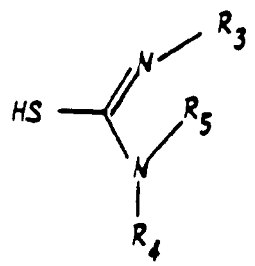

III

$R_1$, $R_2$, $R_3$, $R_4$, $R_5$, A, n et X ont la même signification que précédemment et Y représente un halogène tel que chlore ou brome.

## Patentansprüche

### Patentansprüche für folgende Vertragsstaaten : AT, BE, CH, DE, FR, GB, IT, LI, LU, NL, SE

**1.** Thioformamidine der allgemeinen Formel (I):

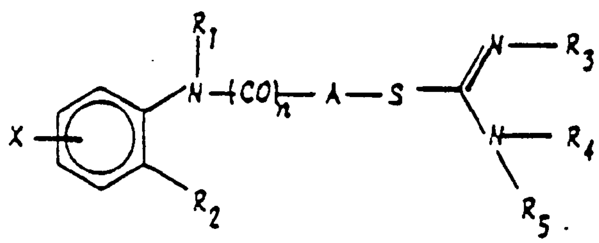

worin bedeuten:
$R_1$ Wasserstoff oder einen niederen Alkylrest mit 1 bis 4 Kohlenstoffatomen;
$R_2$ einen Benzoyl-, Benzyl- oder α-Hydroxy-benzylrest, wobei der aromatische Kern durch ein Halogenatom substituiert sein kann;
$R_3$ und $R_4$, die gleich oder verschieden sind, Wasserstoff, einen Alkylrest oder einen niederen $C_1$-$C_4$-Alkenylrest oder worin $R_3$ und $R_4$ gemeinsam zusammen mit der Formamidin-Funktion einen Imidazol-, Imidazolin-, Benzimidazol-, Triazol-, Pyrimidin-, Tetrahydropyrimidin-Heterocyclus bilden;
$R_5$ Wasserstoff, einen niederen Alkylrest mit 1 bis 4 Kohlenstoffatomen oder worin $R_5$ zusammen mit $R_4$ eine Doppelbindung (-N=$R_4$) bildet im Falle von pseudoaromatischen Heterocyclen;
n 0 oder 1;
A eine lineare oder verzweigte Alkylengruppe mit 1 bis 4 Kohlenstoffatomen;
X Wasserstoff, eine Halogen-, niedere $C_1$-$C_4$-Alkyl-, niedere $C_1$-$C_4$-Alkoxy- oder Nitrogruppe,
und ihre therapeutisch akzeptablen organischen oder Mineralsalze.

**2.** Verbindung nach Anspruch 1, dadurch gekennzeichnet, daß sie ausgewählt wird aus:
N-Methyl-2-[2-(Δ2-imidazolinyl)thio]-2′-o-chlorobenzoyl-4′-chloro-acetanilid-hydrochlorid;
N-Methyl-2-[2-(Δ2-imidazolinyl)thio]-2′-benzoyl-4′-chloroacetanilid -hydrochlorid;
2-[2-(Δ2-Imidazolinyl)thio]-2′-o-chlorobenzoyl-4′-chloroacetanilid-hydrochlorid;
N-Methyl-2-[2-(1-methyl-imidazolyl)thio]-2′-o-chlorobenzoyl-4′-chloro-acetanilid-hydrochlorid;

2-(2-Imidazolylthio)-2'-benzoyl-4'-chloro-acetanilid-hydrochlorid;
2-(2-Imidazolylthio)-2'-o-chlorobenzoyl-4'-chloro-acetanilid-hydrochlorid;
N-Methyl-2'-o-chlorobenzoyl-4'-chloro-2-[(2-imidazolyl)thio]acetanilid-hydrochlorid;
1-(2-Imidazolylthio)-2-[(2-benzoyl-4-chloro)anilino]ethan;
1-[2-(Δ2-Imidazolinyl)-thio]-(2-benzoyl-4-chloro)-2-anilinoethan;
N-Methyl-2'-o-chlorobenzoyl-4'-chloro-2-[1-methyl-2-(Δ2-imidazolinyl )thio]acetanilid-hydrochlorid;
N-Methyl-2'-(o-chloro-α-hydroxybenzyl)-4'-chloro-2-[2-(Δ2-imidazolinyl )thio]acetanilid;
N-Methyl-2'-benzyl-4'-chloro-2-[2-(Δ2-imidazolinyl)thio]acetanilid-hydrochlorid;
N-Methyl-2'-o-chlorobenzoyl-4'-chloro-2-[2-(Δ2-imidazolinyl)thio]propionanilid-citrat;
N-Methyl-2'-benzoyl-2-[2-imidazolylthio]acetanilid;
N-Methyl-2'-benzoyl-4'-chloro-2-(2-imidazolylthio)acetanilid-hydrochlorid;
N-Methyl-2'-o-chlorobenzoyl-4'-chloro-2-isothioureido-acetanilid-hydrochlorid;
N-Methyl-2'-o-chlorobenzoyl-4'-chloro-(2-pyrimidinyl)-2-thioacetanilid ;
N-Methyl-2'-o-chlorobenzoyl-4'-chloro-(1-ethyl-2-imidazolyl)-2-thio-acetanilid-hydrochlorid;
N-Methyl-2'-o-chlorobenzoyl-4'-chloro-(2-imidazolyl)-3-thio-propionanilid;
N-Methyl-2'-benzoyl-4'-nitro-2-[(2-imidazolyl)thio]-acetanilid-hydrochlorid;
N-Methyl-2'-o-fluorobenzoyl-4'-chloro-2-[(2-imidazolyl)thio]acetanilid-hydrochlorid;
N-Methyl-2'-benzoyl-4'-methoxy-2-[(2-imidazolyl)thio]acetanilid-hydrobromid.

3. Verfahren zur Herstellung der chemischen Verbindungen nach einem der Ansprüche 1 und 2, dadurch gekennzeichnet, daß man ein Halogenderivat (II) mit einem Thioformamidin (III) in einem organischen Lösungsmittel wie Methanol oder Ethanol unter Rückfluß behandelt:

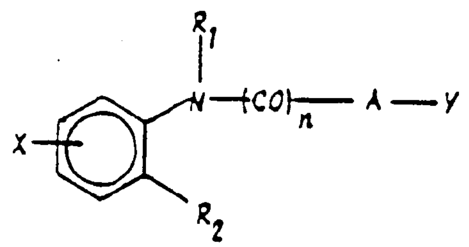

II

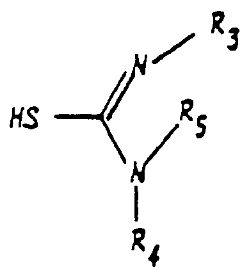

III

worin $R_1$, $R_2$, $R_3$, $R_4$, $R_5$, A, n und X die oben angegebenen Bedeutungen haben und Y ein Halogen wie Chlor oder Brom darstellt.

4. Verbindungen nach einem der Ansprüche 1 und 2 als Arzneimittel, die in der Human- oder Veterinärtherapie verwendbar sind.

5. Verbindungen nach einem der Ansprüche 1 und 2 als Arzneimittel, die für die Behandlung von Geschwüren und die Gastrointestinal-Pathologie verwendbar sind.

6. Pharmazeutische Zusammensetzungen, dadurch gekennzeichnet, daß sie als aktives Prinzip (Wirkstoff) mindestens ein Derivat nach einem der Ansprüche 1 und 2, assoziiert mit einem pharmazeutisch akzeptablen Träger, enthalten.

## Patentansprüche für folgenden Vertragsstaat : ES

1. Verfahren zur Herstellung von Thioformamidinen der allgemeinen Formel (I):

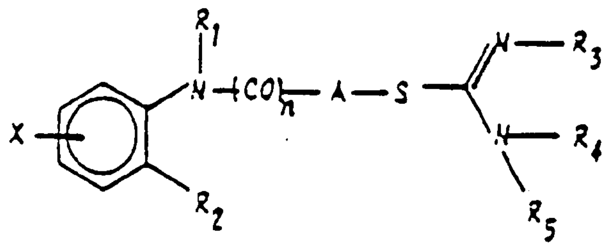

worin bedeuten:
$R_1$ Wasserstoff oder einen niederen Alkylrest mit 1 bis 4 Kohlenstoffatomen;
$R_2$ einen Benzoyl-, Benzyl- oder α-Hydroxy-benzylrest, wobei der aromatische Kern durch ein Halogenatom substituiert sein kann;
$R_3$ und $R_4$, die gleich oder verschieden sind, Wasserstoff, einen Alkylrest oder einen niederen $C_1$-$C_4$-Alkenylrest oder worin $R_3$ und $R_4$ gemeinsam zusammen mit der Formamidin-Funktion einen Imidazol-, Imidazolin-, Benzimidazol-, Triazol-, Pyrimidin-, Tetrahydropyrimidin-Heterocyclus bilden;
$R_5$ Wasserstoff, einen niederen Alkylrest mit 1 bis 4 Kohlenstoffatomen oder worin $R_5$ zusammen mit $R_4$ eine Doppelbindung (-N=$R_4$) bildet im Falle von pseudoaromatischen Heterocyclen;
n 0 oder 1;
A eine lineare oder verzweigte Alkylengruppe mit 1 bis 4 Kohlenstoffatomen;
X Wasserstoff, eine Halogen-, niedere $C_1$-$C_4$-Alkyl-, niedere $C_1$-$C_4$-Alkoxy- oder Nitrogruppe,
und ihrer therapeutisch akzeptablen organischen oder Mineralsalze, dadurch gekennzeichnet, daß man ein Halogenderivat (II) mit einem Thioformamidin (III) in einem organischen Lösungsmittel wie Methanol oder Ethanol unter Rückfluß behandelt:

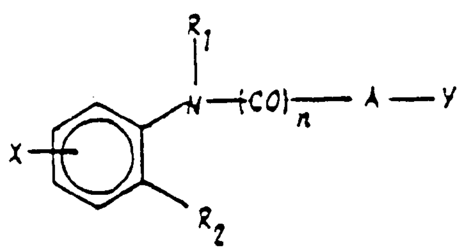

II

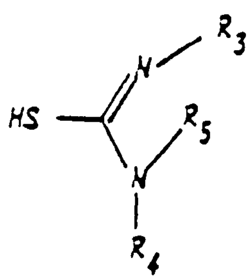

III

worin $R_1$, $R_2$, $R_3$, $R_4$, $R_5$, A, n und X die oben angegebenen Bedeutungen haben und Y ein Halogen wie Chlor oder Brom darstellt.

2. Verfahren nach Anspruch 1, dadurch gekennzeichnet, daß man eine Verbindung herstellt,die ausgewählt wird aus:
N-Methyl-2-[2-(Δ2-imidazolinyl)thio]-2′-o-chlorobenzoyl-4′-chloro-acetanilid-hydrochlorid;
N-Methyl-2-[2-(Δ2-imidazolinyl)thio]-2′-benzoyl-4′-chloroacetanilid-hydrochlorid;
2-[2-(Δ2-Imidazolinyl)thio]-2′-o-chlorobenzoyl-4′-chloroacetanilid-hydrochlorid;
N-Methyl-2-[2-(1-methyl-imidazolyl)thio]-2′-o-chlorobenzoyl-4′-chloro-acetanilid-hydrochlorid;
2-(2-Imidazolylthio)-2′-benzoyl-4′-chloro-acetanilidhydrochlorid;
2-(2-Imidazolylthio)-2′-o-chlorobenzoyl-4′-chloro-acetanilid-hydrochlorid;
N-Methyl-2′-o-chlorobenzoyl-4′-chloro-2-[(2-imidazolyl)thio]acetanilid-hydrochlorid;
1-(2-Imidazolylthio)-2-[(2-benzoyl-4-chloro)anilino]ethan;

1-[2-(Δ2-Imidazolinyl)-thio]-(2-benzoyl-4-chloro)-2-anilinoethan;
N-Methyl-2′-o-chlorobenzoyl-4′-chloro-2-[1-methyl-2(Δ2-imidazolinyl )thio]acetanilid-hydrochlorid;
N-Methyl-2′-(o-chloro-α-hydroxybenzyl)-4′-chloro-2-[2-(Δ2-imidazolinyl )thio]acetanilid;
N-Methyl-2′-benzyl-4′-chloro-2-[2-(Δ2-imidazolinyl)thio]acetanilid-hydrochlorid;
N-Methyl-2′-o-chlorobenzoyl-4′-chloro-2-[2-(Δ2-imidazolinyl)thio]propionanilid-citrat;
N-Methyl-2′-benzoyl-2-[2-imidazolylthio]acetanilid;
N-Methyl-2′-benzoyl-4′-chloro-2-(2-imidazolylthio)acetanilid-hydrochlorid;
N-Methyl-2′-o-chlorobenzoyl-4′-chloro-2-isothioureido-acetanilid-hydrochlorid;
N-Methyl-2′-o-chlorobenzoyl-4′-chloro-(2-pyrimidinyl)-2-thioacetanilid ;
N-Methyl-2’-o-chlorobenzoyl-4’-chloro-(1-ethyl-2-imidazolyl)-2-thio-acetanilid-hydrochlorid;
N-Methyl-2′-o-chlorobenzoyl-4′-chloro-(2-imidazolyl)-3-thio -propionanilid;
N-Methyl-2′-benzoyl-4′-nitro-2-[(2-imidazolyl)thio]-acetanilid-hydrochlorid;
N-Methyl-2′-o-fluorobenzoyl-4′-chloro-2-[(2-imidazolyl)thio]cetanilid-hydrochlorid;
N-Methyl-2′-benzoyl-4’-methoxy-2-[(2-imidazolyl)thio]acetanilid-hydrobromid.

**Patentansprüche für folgenden Vertragsstaat : GR**

**1.** Thioformamidine der allgemeinen Formel (I):

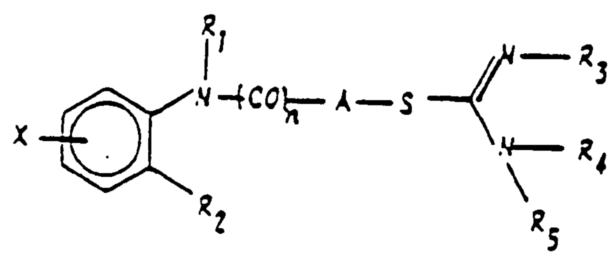

worin bedeuten:
$R_1$ Wasserstoff oder einen niederen Alkylrest mit 1 bis 4 Kohlenstoffatomen;
$R_2$ einen Benzoyl-, Benzyl- oder α-Hydroxy-benzylrest, wobei der aromatische Kern durch ein Halogenatom substituiert sein kann;
$R_3$ und $R_4$, die gleich oder verschieden sind, Wasserstoff, einen Alkylrest oder einen niederen $C_1$-$C_4$-Alkenylrest oder worin $R_3$ und $R_4$ gemeinsam zusammen mit der Formamidin-Funktion einen Imidazol-, Imidazolin-, Benzimidazol-, Triazol-, Pyrimidin-, Tetrahydropyrimidin-Heterocyclus bilden;
$R_5$ Wasserstoff, einen niederen Alkylrest mit 1 bis 4 Kohlenstoffatomen oder worin $R_5$ zusammen mit $R_4$ eine Doppelbindung (-N=$R_4$) bildet im Falle von pseudoaromatischen Heterocyclen;
n 0 oder 1;
A eine lineare oder verzweigte Alkylengruppe mit 1 bis 4 Kohlenstoffatomen;
X Wasserstoff, eine Halogen-, niedere $C_1$-$C_4$-Alkyl-, niedere $C_1$-$C_4$-Alkoxy- oder Nitrogruppe,
und ihre therapeutisch akzeptablen organischen oder Mineralsalze.

**2.** Verbindung nach Anspruch 1, dadurch gekennzeichnet, daß sie ausgewählt wird aus:
N-Methyl-2-[2-(Δ2-imidazolinyl)thio]-2′-o-chlorobenzoyl-4 ′-chloro-acetanilid-hydrochlorid;
N-Methyl-2-[2-(Δ2-imidazolinyl)thio]-2′-benzoyl-4′-chloroacetanilid-hydrochlorid;
2-[2-(Δ2-Imidazolinyl)thio]-2′-o-chlorobenzoyl-4′-chloroacetanilid-hydrochlorid;
N-Methyl-2-[2-(1-methyl-imidazolyl)thio]-2′-o-chlorobenzoyl-4′-chloro-acetanilid-hydrochlorid;
2-(2-Imidazolylthio)-2′-benzoyl-4′-chloro-acetanilid-hydrochlorid;
2-(2-Imidazolylthio)-2′-o-chlorobenzoyl-4′-chloro-acetanilid-hydrochlorid;
N-Methyl-2′-o-chlorobenzoyl-4′-chloro-2-[(2-imidazolyl)thio]acetanilid-hydrochlorid;
1-(2-Imidazolylthio)-2-[(2-benzoyl-4-chloro)anilino]ethan;
1-[2-(Δ2-Imidazolinyl)-thio]-(2-benzoyl-4-chloro)-2-anilinoethan;
N-Methyl-2′-o-chlorobenzoyl-4′-chloro-2-[1-methyl-2-(Δ2-imidazolinyl )thio]acetanilid-hydrochlorid;
N-Methyl-2′-(o-chloro-α-hydroxybenzyl)-4′-chloro-2-[2-Δ2-imidazolinyl )thio]acetanilid;
N-Methyl-2′-benzyl-4′-chloro-2-[2-(Δ2-imidazolinyl)thio]acetanilid-hydrochlorid;
N-Methyl-2′-o-chlorobenzoyl-4′-chloro-2-[2-(Δ2-imidazolinyl)thio]propionanilid-citrat;
N-Methyl-2′-benzoyl-2-[2-imidazolylthio]acetanilid;

N-Methyl-2′-benzoyl-4′-chloro-2-(2-imidazolylthio)acetanilid-hydrochlorid;
N-Methyl-2′-o-chlorobenzoyl-4′-chloro-2-isothioureido-acetanilid-hydrochlorid;
N-Methyl-2′-o-chlorobenzoyl-4′-chloro-(2-pyrimidinyl)-2-thioacetanilid ;
N-Methyl-2′-o-chlorobenzoyl-4′-chloro-(1-ethyl-2-imidazolyl)-2-thio-acetanilid-hydrochlorid;
N-Methyl-2′-o-chlorobenzoyl-4′-chloro-(2-imidazolyl)-3-thio-propionanilid;
N-Methyl-2′-benzoyl-4′-nitro-2-[(2-imidazolyl)thio)-acetanilid-hydrochlorid;
N-Methyl-2′-o-fluorobenzoyl-4′-chloro-2-[(2-imidazolyl)thio)acetanilid-hydrochlorid;
N-Methyl-2′-benzoyl-4′-methoxy-2-[(2-imidazolyl)thio)acetanilid-hydrobromid.

3. Verfahren zur Herstellung der chemischen Verbindungen nach einem der Ansprüche 1 und 2, dadurch gekennzeichnet, daß man ein halogeniertes Derivat (II) mit einem Thioformamidin (III) in einem organischen Lösungsmittel wie Methanol oder Ethanol unter Rückfluß behandelt:

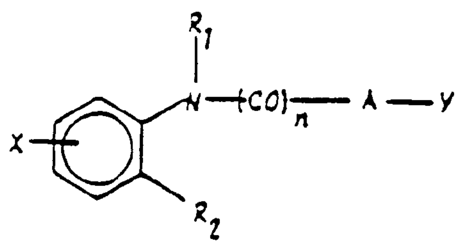

II

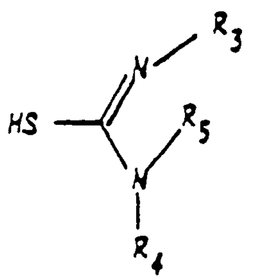

III

worin $R_1$, $R_2$, $R_3$, $R_4$, $R_5$, A, n und X die oben angegebenen Bedeutungen haben und Y ein Halogen wie Chlor oder Brom darstellt.

## Claims

### Claims for the following Contracting States : AT, BE, CH, DE, FR, GB, IT, LI, LU, NL, SE

1. Thioformamidines corresponding to the general formula I :

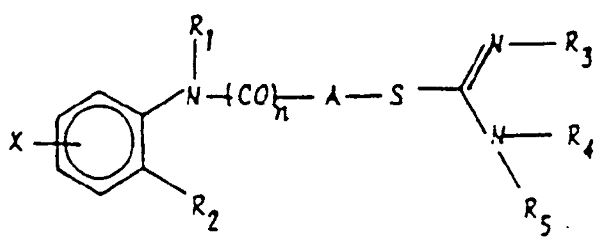

in which :
- $R_1$ represents hydrogen or a lower alkyl radical containing one to four carbon atoms ;
- $R_2$ represents a benzoyl, benzyl or a hydroxy benzyl radical, the aromatic nucleus being capable of substitution with a halogen atom ;
- $R_3$ and $R_4$, which are identical to, or different from, each other, represent hydrogen, an alkyl or alkenyl radical lower than $C_{14}$, or form between them with the formamidine group a heterocyclic imidazole, imidazoline, benzimidazole, triazole, pyrimidine or tetrahydropyrimidine ;

- $R_5$ represents hydrogen or a lower alkyl radical containing 1-4 carbon atoms or forms a double bond with $R_4$ ($-N=R_4$) in the case of pseudoaromatic heterocyclics ;
- n = 0 or 1 ;
- A represents a linear or branched alkylene group, having from 1-4 carbon atoms ;
- X represents hydrogen, a halogen, an alkyl lower than $C_{1-4}$, an alkoxy lower than $C_{1-4}$ or a nitro group,

and their therapeutically acceptable salts with organic or inorganic acids.

**2.** A compound according to Claim 1 characterized in that it is chosen from among the following :
- N-methyl [(Δ2-imidazolinyl)-2 thio]-2 orthochlorobenzoyl-2′ chloro-4′ acetanilide hydrochloride ;
- N-methyl [(Δ2-imidazolinyl)-2 thio]-2 benzoyl-2′ chloro-4′ acetanilide hydrochloride ;
- [(Δ2-imidazolinyl)-2 thio]-2 orthochlorobenzoyl-2′ chloro-4′ acetanilide hydrochloride ;
- N-methyl [(methyl-1 imidazolyl)-2 thio]-2 orthochlorobenzoyl-2′ chloro-4′ acetanilide hydrochloride ;
- (imidazolyl-2 thio)-2 benzoyl-2′ chloro-4′ acetanilide hydrochloride ;
- (imidazolyl-2 thio)-2 orthochlorobenzoyl-2′ chloro-4′ acetanilide hydrochloride ;
- N-methyl o. chlorobenzoyl-2′ chloro-4′ [(imidazolyl-2) thio]-2 acetanilide hydrochloride ;
- (imidazolyl-2 thio)-1 [(benzoyl-2 chloro-4) anilino]-2 ethane;
- [(Δ2-imidazolinyl)-2 thio]-1 (benzoyl-2 chloro-4) anilino-2 ethane ;
- N-methyl o.chlorobenzoyl-2′ chloro-4′ [methyl-1 Δ2-imidazolinyl)-2 thio]-2 acetanilide hydrochloride ;
- N-methyl (orthochloro α-hydroxybenzyl)-2′ chloro-4′ [Δ2-imidazolinyl)-2 thio]-2 acetanilide ;
- N-methyl benzyl-2′ chloro-4′ [(Δ2-imidazolinyl)-2 thio]-2 acetanilide hydrochloride ;
- N-methyl orthochlorobenzoyl-2′ chloro-4′ [(Δ2-imidazolinyl)-2 thio]-2 propionanilide citrate ;
- N-methyl benzoyl-2′ [imidazolyl-2 thio]-2 acetanilide ;
- N-methyl benzoyl-2′ chloro-4′ (imidazolyl-2 thio)-2 acetanilide hydrochloride ;
- N-methyl orthochlorobenzoyl-2′ chloro-4′ isothioureido-2 acetanilide hydrochloride ;
- N-methyl orthochlorobenzoyl-2′ chloro-4′ (pyrimidinyl-2) thio-2 acetanilide ;
- N-methyl orthochlorobenzoyl-2′ chloro-4′ (ethyl-1 imidazolyl-2) thio-2 acetanilide hydrochloride ;
- N-methyl orthochlorobenzoyl-2′ chloro-4′ (imidazolyl-2) thio-3 propionanilide ;
- N-methyl benzoyl-2′ nitro-4′ [(imidazolyl-2) thio]-2 acetanilide hydrochloride ;
- N-methyl o.fluorobenzoyl-2′ chloro-4′ [(imidazolyl-2) thio]-2 acetanilide hydrochloride ;
- N-methyl benzoyl-2′ methoxy-4′ [(imidazolyl-2) thio]-2 acetanilide hydrobromide ;

**3.** A process for preparing chemical compounds according to either of the Claims 1 and 2 characterized in that a halogen derivative II is treated with a thioformamidine III under reflux with an organic solvent such as methanol or ethanol.

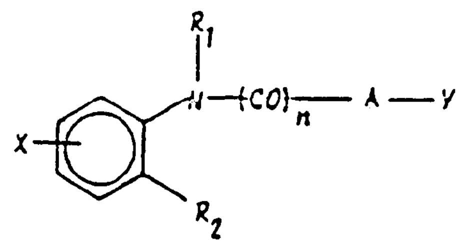

II

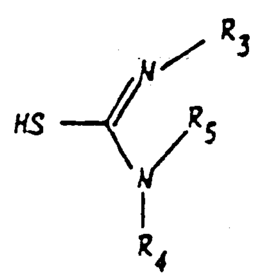

III

$R_1$, $R_2$, $R_3$, $R_4$, $R_5$, A, n and X have the same meaning as previously and Y represents a halogen such as chlorine or bromine.

**4.** For medications which are useful in human or veterinary therapy, compounds according to either of Claims

1 and 2.

5. For medications which are useful in the treatment of ulcers and in gastro-intestinal pathology, compounds according to either of Claims 1 and 2.

6. Pharmaceutical compositions characterized in that they contain, as the active principle, at least one derivative according to either of Claims 1 and 2, associated with a pharmaceutically acceptable base.

**Claims for the following Contracting State : ES**

1. Process for the preparation of thioformamidines corresponding to the general formula I :

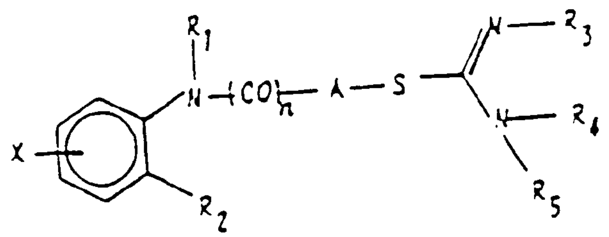

in which :
- $R_1$ represents hydrogen or a lower alkyl radical containing one to four carbon atoms ;
- $R_2$ represents a benzoyl, benzyl or a hydroxy benzyl radical, the aromatic nucleus being capable of substitution with a halogen atom ;
- $R_3$ and $R_4$, which are identical to, or different from, each other, represent hydrogen, an alkyl or alkenyl radical lower than $C_{1-4}$, or form between them with the formamidine group a heterocyclic imidazole, imidazoline, benzimidazole, triazole, pyrimidine or tetrahydropyrimidine ;
- $R_5$ represents hydrogen or a lower alkyl radical containing 1-4 carbon atoms or forms a double bond with $R_4$ (-N=$R_4$) in the case of pseudo aromatic heterocyclics ;
- n = 0 or 1 ;
- A represents a linear or branched alkylene group, having from 1-4 carbon atoms ;
- X represents hydrogen, a halogen, an alkyl lower than $C_{1-4}$, an alkoxy lower than $C_{1-4}$ or a nitro group,

and their therapeutically acceptable salts with organic or inorganic acids, characterized in that a halogen derivative II is treated with a formamidine III under reflux with an organic solvent such as methanol or ethanol.

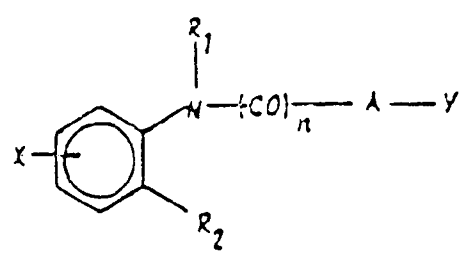

II

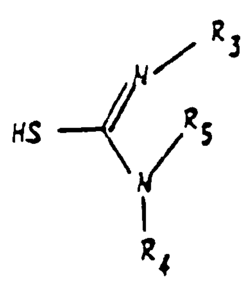

III

$R_1$, $R_2$, $R_3$, $R_4$, $R_5$, A, n and X have the same meaning as previously and Y represents a halogen such as

chlorine or bromine.

2. Process according to Claim 1, characterized in that a compound is prepared chosen from among :
   - N-methyl [(Δ2-imidazolinyl)-2 thio]-2 orthochlorobenzoyl-2′ chloro-4′ acetanilide hydrochloride ;
   - N-methyl [(Δ2-imidazolinyl)-2 thio]-2 benzoyl-2′ chloro-4 ′ acetanilide hydrochloride ;
   - [(Δ2-imidazolinyl)-2 thio]-2 orthochlorobenzoyl-2′ chloro-4′ acetanilide hydrochloride ;
   - N-methyl [(methyl-1 imidazolyl)-2 thio]-2 orthochlorobenzoyl-2′ chloro-4′ acetanilide hydrochloride ;
   - (imidazolyl-2 thio)-2 benzoyl-2′ chloro-4′ acetanilide hydrochloride ;
   - (imidazolyl-2 thio)-2 orthochlorobenzoyl-2′ chloro-4′ acetanilide hydrochloride ;
   - N-methyl o. chlorobenzoyl-2′ chloro-4′ [(imidazolyl-2) thio]-2 acetanilide hydrochloride ;
   - (imidazolyl-2 thio)-1 [(benzoyl-2 chloro-4) anilino]-2 ethane;
   - [(Δ2-imidazolinyl)-2 thio]-1 (benzoyl-2 chloro-4) anilino-2 ethane ;
   - N-methyl o.chlorobenzoyl-2′ chloro-4′ [methyl-1 Δ2-imidazolinyl)-2 thio]-2 acetanilide hydrochloride ;
   - N-methyl (orthochloro α-hydroxybenzyl)-2′ chloro-4′ [(Δ2-imidazolinyl)-2 thio]-2 acetanilide ;
   - N-methyl benzyl-2′ chloro-4′ [(Δ2-imidazolinyl)-2 thio]-2 acetanilide hydrochloride ;
   - N-methyl orthochlorobenzoyl-2′ chloro-4′ [(Δ2-imidazolinyl)-2 thio]-2 propionanilide citrate ;
   - N-methyl benzoyl-2′ [imidazolyl-2 thio]-2 acetanilide ;
   - N-methyl benzoyl-2′ chloro-4′ (imidazolyl-2 thio)-2 acetanilide hydrochloride ;
   - N-methyl orthochlorobenzoyl-2′ chloro-4′ isothioureido-2 acetanilide hydrochloride ;
   - N-methyl orthochlorobenzoyl-2′ chloro-4′ (pyrimidinyl-2) thio-2 acetanilide ;
   - N-methyl orthochlorobenzoyl-2′ chloro-4′ (ethyl-1 imidazolyl-2) thio-2 acetanilide hydrochloride ;
   - N-methyl orthochlorobenzoyl-2′ chloro-4′ (imidazolyl-2) thio-3 propionanilide ;
   - N-methyl benzoyl-2′ nitro-4′ [(imidazolyl-2) thio]-2 acetanilide hydrochloride ;
   - N-methyl o.fluorobenzoyl-2′ chloro-4′ [(imidazolyl-2) thio]-2 acetanilide hydrochloride ;
   - N-methyl benzoyl-2′ methoxy-4′ [(imidazolyl-2) thio]-2 acetanilide hydrobromide.

**Claims for the following Contracting State : GR**

1. Thioformamidines corresponding to the general formula I :

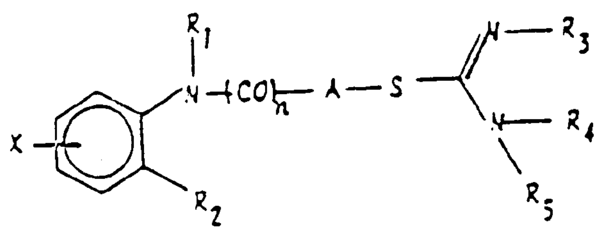

in which :
   - $R_1$ represents hydrogen or a lower alkyl radical containing one to four carbon atoms ;
   - $R_2$ represents a benzoyl, benzyl or α hydroxy benzyl radical, the aromatic nucleus being capable of substitution with a halogen atom ;
   - $R_3$ and $R_4$, which are identical to, or different from, each other, represent hydrogen, an alkyl or alkenyl radical lower than $C_{1-4}$, or form between them with the formamidine group a heterocyclic imidazole, imidazoline, benzimidazole, triazole, pyrimidine or tetrahydropyrimidine ;
   - $R_5$ represents hydrogen or a lower alkyl radical containing 1-4 carbon atoms or forms a double bond with $R_4$ ($-N=R_4$) in the case of pseudoaromatic heterocyclics ;
   - n = 0 or 1 ;
   - A represents a linear or branched alkylene group, having from 1-4 carbon atoms ;
   - X represents hydrogen, a halogen, an alkyl lower than $C_{1-4}$, an alkoxy lower than $C_{1-4}$ or a nitro group,

   and their therapeutically acceptable salts with organic or inorganic acids.

2. a compound according to Claim 1 characterized in that it is chosen from among the following :
   - N-methyl [(Δ2-imidazolinyl)-2 thio]-2 orthochlorobenzoyl-2′ chloro-4′ acetanilide hydrochloride ;
   - N-methyl [(Δ2-imidazolinyl)-2 thio]-2 benzoyl-2′ chloro-4 ′ acetanilide hydrochloride ;
   - [(Δ2-imidazolinyl)-2 thio]-2 orthochlorobenzoyl-2′ chloro-4′ acetanilide hydrochloride ;
   - N-methyl [(methyl-1 imidazolyl)-2 thio]-2 orthochlorobenzoyl-2′ chloro-4′ acetanilide hydrochloride ;

- (imidazolyl-2 thio)-2 benzoyl-2′ chloro-4′ acetanilide hydrochloride ;
- (imidazolyl-2 thio)-2 orthochlorobenzoyl-2′ chloro-4′ acetanilide hydrochloride ;
- N-methyl o. chlorobenzoyl-2′ chloro-4′ [(imidazolyl-2) thio]-2 acetanilide hydrochloride ;
- (imidazolyl-2 thio)-1 [(benzoyl-2 chloro-4) anilino]-2 ethane;
- [(Δ2-imidazolinyl)-2 thio]-1 (benzoyl-2 chloro-4) anilino-2 ethane ;
- N-methyl o.chlorobenzoyl-2′ chloro-4′ [methyl-1 Δ2-imidazolinyl)-2 thio]-2 acetanilide hydrochloride ;
- N-methyl (orthochloro α-hydroxybenzyl)-2′ chloro-4′ [Δ2-imidazolinyl)-2 thio]-2 acetanilide ;
- N-methyl benzyl-2′ chloro-4′ [(Δ2-imidazolinyl)-2 thio]-2 acetanilide hydrochloride ;
- N-methyl orthochlorobenzoyl-2′ chloro-4′ [(Δ2-imidazolinyl)-2 thio]-2 propionanilide citrate ;
- N-methyl benzoyl-2′ [imidazolyl-2 thio]-2 acetanilide ;
- N-methyl benzoyl-2′ chloro-4′ (imidazolyl-2 thio)-2 acetanilide hydrochloride ;
- N-methyl orthochlorobenzoyl-2′ chloro-4′ isothioureido-2 acetanilide hydrochloride ;
- N-methyl orthochlorobenzoyl-2′ chloro-4′ (pyrimidinyl-2) thio-2 acetanilide ;
- N-methyl orthochlorobenzoyl-2′ chloro-4′ (ethyl-1 imidazolyl-2) thio-2 acetanilide hydrochloride ;
- N-methyl orthochlorobenzoyl-2′ chloro-4′ (imidazolyl-2) thio-3 propionanilide ;
- N-methyl benzoyl-2′ nitro-4′ [(imidazolyl-2) thio]-2 acetanilide hydrochloride ;
- N-methyl o.fluorobenzoyl-2′ chloro-4′ [(imidazolyl-2) thio]-2 acetanilide hydrochloride ;
- N-methyl benzoyl-2′ methoxy-4′ [(imidazolyl-2) thio]-2 acetanilide hydrobromide ;

**3.** A process for preparing chemical compounds according to either of the Claims 1 and 2 characterized in that a halogen derivative II is treated with a thioformamidine III under reflux with an organic solvent such as methanol or ethanol.

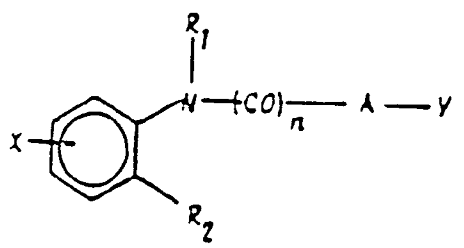

II

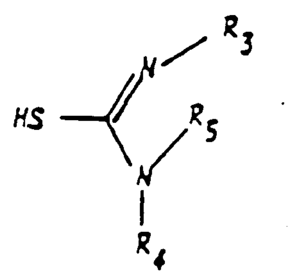

III

$R_1$, $R_2$, $R_3$, $R_4$, $R_5$, A, n and X have the same meaning as previously and Y represents a halogen such as chlorine or bromine.